# EUROPEAN PATENT APPLICATION

(11) **EP 4 434 554 A1**
(43) Date of publication of application: **25.09.2024**
(21) Application number: 23163791.9
(22) Date of filing: 23.03.2023
(51) Int. Cl.: A61M 1/06, G01N 21/3577, G01N 33/04, G01N 33/487, A01J 5/007

(54) **HUMAN MILK HANDLING APPARATUS**

(71) Applicant: Medela Holding AG, 6340 Baar (CH)
(72) Inventor: Thüring, Martin, 5634 Merenschwand (CH); Höner, Sebastian, 8800 Thalwil (CH)
(74) Representative: Grünecker Patent- und Rechtsanwälte PartG mbB

(57) **Abstract**

The disclosed invention pertains to a human milk handling apparatus (1) comprising a measuring device (4) that has a sensor unit (15) for emitting light (L) towards human milk (M) and for detecting light reflected by or transmitted through the human milk (M); and comprising a processing unit which is adapted to determine at least a component content of said human milk (M) and connected to said sensor unit (15), said sensor unit (15) being adapted to emit and/or detect the light (L) at at least one specific wavelength.

## Description

The present invention relates to a human milk handling apparatus. The apparatus comprises a measuring device that has a sensor unit and a processing unit connected to the sensor unit.

The present invention further relates to a pump and a method, particularly to a breast pump built to express human milk from a lactating mother, to a milk pump built to feed a newborn with human milk, and to a method for analyzing human milk.

EP3610900A1 discloses an apparatus where light is sent to milk in a reservoir and where the light is measured using an array of light barriers. A sensor unit for the measurement is in a detachable assembly and in milk contact.

WO2021165281A1 discloses an apparatus where light is sent to milk in a reservoir and where the light is guided by mirror structures. A sensor unit for the measurement is in a detachable assembly and is not in milk contact.

EP3536359A1 discloses an apparatus with a flow sensor for milk in a combination with a siphon. The flow sensor may be an optical sensor.

The documents known from prior art teach to measure the flow and/or volume of the milk expressed from the breast of a mother, but none of them address milk composition like fat content and/or protein content of the milk.

It is an object of the present invention to provide an apparatus and a method which provide a measurement of at least a milk component content.

According to the invention, an apparatus comprising the features of claim 1, a breast pump comprising the features of claim 11 and a method comprising the features of claim 12 are suggested. Preferred embodiments are given in the dependent claims.

As a solution to this problem, a human milk handling apparatus is suggested that comprises a measuring device, particularly to be coupled to a milk container, e.g. of a pump or of the apparatus, and more particularly having a connector for the pump. The measuring device, particularly a measuring head thereof, comprises a sensor unit for emitting light towards human milk and for detecting said light particularly reflected by and/or transmitted through the human milk, and a processing unit which is adapted to determine a component content of said human milk and which is connected to the sensor unit.

To particularly enhance the measurement, the sensor unit is adapted to emit the light at at least one specific wavelength, to detect the light at at least one specific wavelength, or to emit and detect the light at at least one specific wavelength. The at least one specific wavelength pertains to one, two, three or more specific wavelength(s), and particular to a wavelength of emission and/or a wavelength of detection. In other words, the sensor unit may emit at a certain wavelength, and additionally or alternatively the sensor unit may detect at said certain wavelength or at another one.

When the sensor unit is adapted to emit and/or detect the light at at least one specific wavelength, the sensor unit is substantially focused by means of hardware and/or software towards this particular at least one specific wavelength. This means that at a wavelength different from the at least one specific wavelength, the emission and/or detection at least essentially is not possible, not realized and/or not allowed as per the corresponding manufacturer's data sheet or the corresponding manufacturer's recommendation. Particularly, the emission and/or detection has its substantial peak at the at least one specific wavelength.

Preferably, "at least one/two/three/etc. specific wavelength" may as well be realized as "exactly one/two/three/etc. specific wavelength", with "exactly" meaning "not more and not less than", in order to facilitate the measurement at low cost.

Essentially an apparatus is suggested that is particularly meant for a pump, e.g. in the form of a breast pump used to express human milk (in short: "milk") from a lactating mother, or in the form a milk pump used to feed a newborn with human milk, or another pump in the context of human milk handling. The apparatus may have a reservoir to accumulate the human milk in order to determine the component content, wherein the light can be emitted towards and/or reflected from the milk. As such, the sensor unit typically at least partially points towards the reservoir with the human milk so that a determination of the component content can take place. The apparatus is typically built to collect milk in a reservoir, where the reservoir may be a reservoir of the pump or of the apparatus, particularly the milk may additionally be collected in a milk container. The pump or the apparatus may comprise the milk container, particularly wherein the milk container is larger than the reservoir and/or is for accumulating the milk subsequent to the reservoir and/or separately therefrom. The apparatus may have a connector for the pump, particularly for a suction source. The apparatus can be connected to the pump and has a measuring device. The measuring device may be connected and/or designed to be attached to the milk container. Particularly, the measuring device has a sensor unit and a processing unit, particularly a measuring head therewith, wherein the sensor unit is typically configured to provide at least one sensor signal to the processing unit. If provided, the reservoir may be part of the measuring device and/or the measuring head. The sensor unit is preferably built to emit/send light towards the human milk, and to receive/detect light, particularly from the human milk.

The processing unit may receive the at least one sensor signal from the sensor unit particularly to conduct a measurement with respect to the milk and/or to determine the component content of the milk. The component content of the milk is meant to provide information about or represent physical and/or chemical characteristics of the milk, e.g. the amount of milk, particularly in the reservoir, and/or a nutritional fact about the milk, such as fat content. The component content of the milk may be a physical and/or chemical one.

What has been found to be very beneficial towards a more precise measurement of the milk is named herein, namely "the sensor unit is adapted to emit and/or detect the light at at least one specific wavelength", the features which may be formulated in other words as follows: the sensor unit may be adapted to generate and/or receive the light, where the light may be generated with one, two or more specific wavelength(s) and/or where the reception of the light is conducted with one, two or more specific wavelength(s). In the case of two or more specific wavelength(s) (i.e. "the at least one specific wavelength" pertains to a plurality of wavelengths or wavelength values), the specific wavelengths shall be different in their absolute values.

The invention provides that the milk may be estimated in terms of its components, for example fat/water/lactose/protein content, macro nutrients and/or milk components. The invention and the preferred embodiments may enable a simple and cheap analysis of the milk.

The milk may be estimated particularly with respect to a fat content of the milk. A measured transmittance and/or reflectance (e.g. the intensity of the light transmitted/reflected relative to the intensity of the light originally emitted) at the milk (or at the reservoir containing the milk) is highly dependent on the fat content of the milk. Thus, the fat content may be estimated by means of the processing unit handling the sensor unit, particularly considering the sensor signal received therefrom.

The invention is preferably used when human milk is handled. As such, human milk may be pumped by means of a pump. The pump, particularly in the form of a breast pump or a milk pump, typically serves handle milk at least in the sense of pumping milk, particularly from a human breast and/or to a newborn. The pump may be able to provide a vacuum. The pump may comprise a vacuum pump. A vacuum may be used at the human breast in order to pump milk. Particularly, milk at the beginning of a pumping session can be different than in the middle and/or at the end of a pumping session. The milk at the beginning of a pumping session may be very translucent and/or watery relative to the middle and/or end of the pumping session. The milk may get significantly less translucent and/or less watery over the time of the session after the beginning. For example, the beginning of the pumping session may last between 1 second and 400 seconds, and the middle of the pumping session may follow after the beginning. The overall pumping session may last between 900 to 1500 seconds.

The transmissive and/or blocking behavior of milk with respect to light depends on the light's wavelength. It is understood that at specific wavelengths the transmissive and/or blocking behavior of the milk is more depending on the composition of the milk, e.g. fat or protein content, relative to other wavelengths that are different from the specific wavelengths. Therefore, a component content of the milk may be estimated by means of a measurement that depends on a transmission of light through the milk.

The reflective behavior of milk with respect to light depends on the light's wavelength. It is understood that at specific wavelengths the reflective behavior of the milk is more depending on the composition of the milk, e.g. fat or protein content, relative to other wavelengths that are different from the at least one specific wavelength. Therefore, the component content of the milk may be estimated by means of a measurement that depends on a reflection of light from the milk.

As such, the transmissive/blocking behavior and the reflective behavior may complement each other in order to estimate the component content or the content of more com ponents of the milk.

The processing unit may estimate the component content of the milk, such as the fat content. Other estimations are anyhow possible for the milk content, e.g. protein content, lactose content, or even others.

The specific wavelengths are particularly understood as specific values in the range of 0 nm to 2500 nm, e.g. accompanied by an uncertainty of up to 1 %, 2 % or more.

The measuring head and/or the measuring device may comprise or consist of a housing. The measuring head may be shaped in order to be attached and/or detached from the measuring device, for example by means of a clamp at the measuring head. The measuring device and/or the measuring head may be shaped in order to be attached and/or detached from the milk container, for example by means of a clamp at the measuring device and/or the measuring head. The measuring head and/or the measuring device may support and/or hold the reservoir, the sensor unit and/or the processing unit. The measuring head and/or the measuring device may - at least partially - be transparent to the light, particularly at the at least one specific wavelength.

It may be the case that the at least one specific wavelength comprises a wavelength of emission (related to the light emission) and a wavelength of detection (related to the light detection) which are at least substantially the same. In other words, the emission and the detection may take place at at least substantially the same wavelength values. At least substantially the same may include a deviation around one particular value in the range of up to 10 %, 5 %, 2,5 %, 1 % or less. This may constrain the particular parts, hardware and/or software needed, e.g. make it cheaper, and may enable an enhanced component content estimation.

The sensor unit may comprise at least one light emitter which is built to emit light at the at least one specific wavelength, particularly at at least one of the at least two specific wavelengths. There may be another light emitter which is built to emit light at another specific wavelengths of the at least one specific wavelength, particularly of the at least two specific wavelengths.

The sensor unit may comprise at least one light detector which detects light at at least one of the at least one specific wavelength. There may be another light detector to detect light at another specific wavelengths of the at least one specific wavelength.

In particular, the light emitter may emit light at two different specific wavelengths. Alternatively, the light emitter may emit light at only one specific wavelength, where another light emitter may emit light at another specific wavelength.

In particular, the light detector may detect light at two different specific wavelengths. Alternatively, the light detector may detect light at only one specific wavelength, where another light detector may detect light at another specific wavelength.

The at least one specific wavelength may be above 400 nm and/or below 1000 nm.

The at least one specific wavelength may be above 400 nm and/or below 1100 nm.

The at least one specific wavelength may be contained in a first range between 920 nm and 960 nm and/or in a second range between 750 nm and 800 nm and/or in a third range between 1040 nm and 1090 nm. For example, at 940 nm ± 1 % fat (content) may be analyzed. At 776 nm ± 1 % protein (content) may be analyzed. At 1064 nm ± 1% lactose (content) may be analyzed. The range of ± 1 % may as well be selected larger or smaller, e.g. 0,5 %, 2 %, 3 %, 4 % or 5 %. Such wavelengths/ranges have been proven to result in a good estimation of the component content of the milk.

One of the at least one light emitter may be a reflectance light emitter and one of the at least one light detector may be a reflectance light detector. Said emitter and said detector may be arranged on the same side of the milk. The reflectance light emitter may be particularly adapted to emit light to be reflected by the milk. The reflectance light detector may be particularly adapted to receive said reflected light. Particularly, said emitter(s) and detector(s) may be placed next to each other and/or facing at least essentially towards the same direction or at a slight angle for the reflection to be accordingly received.

One of the at least one light emitter may be a transmittance light emitter and one of the at least one light detector may be a transmittance light detector. Said emitter and said detector may be arranged on opposing sides of the milk and/or the reservoir. The transmittance light emitter may be particularly adapted to emit light to be transmitted through the milk container, the reservoir and/or the milk. The transmittance light detector may be particularly adapted to receive said transmitted light. Particularly, said emitter(s) and detector(s) may be placed facing each other and/or opposite from each other for the transmittance to be accordingly received.

The processing unit may be adapted to determine at least one of the following component content: macro nutrients, fat content, protein content, lactose content and components of the human milk. As such, the processing unit may be trained and/or using training data. The processing unit may conduct a correlation in order to determine said component content(s).

Preferably the processing unit is adapted to process at least one sensor signal or two or more sensor signals from the sensor unit in order to determine the component content(s). Preferably, the sensor signal or each of the two or more sensor signals pertains to one of the at least one specific wavelength, particularly where at said wavelength either the emission takes place, the detection takes place, or both.

The sensor unit, particularly the measuring device, may be adapted to detect an amount of milk, for example in the reservoir. This may be realized by placing the emitter(s) and detector(s) at certain fill level(s), particularly relative to the reservoir. The electromagnetic light may be reflected and/or further transmitted towards the detector as a function of the presence of milk in front of the emitter. The processing unit may be adapted accordingly to determine or estimate the amount of milk. As such, both the composition and the fill level and/or milk flow may be measured, in particular simultaneously.

The arrangement may be realized by a placement of emitter(s) and detector(s) in front of each other or next to each other so that the milk is reflected or transmitted as a function of the presence of milk in front of or between the emitter(s) and detector(s).

Further, a method is suggested. The method is meant for analyzing human milk, preferably in an apparatus or a pump, such as the apparatus or the pump described herein. In the method light preferably is emitted towards the human milk, wherein the light preferably is reflected at and/or transmitted through the human milk, wherein the light preferably is detected, and/or wherein the emission and/or the detection of the light preferably takes place at at least one specific wavelength.

Preferably, the at least one specific wavelength contains a wavelength of transmission and a wavelength of detection which are are at least substantially the same.

By means of the method, one or also more the one component content of the milk may be determined or estimated, e.g. to get to know the fat or protein content of the milk.

Sensor signals resulting from a reflectance measurement or a transmissive measurement or a combination thereof may be obtained. The signals may be electronically processed to output information of at least one component content of the milk.

Preferably the component content of the milk is selected from the group comprising or consisting of macro nutrients, fat content, protein content and/or other components of the milk.

Especially for conducting the method, the sensor unit may be configured to provide the at least one, particularly two or more sensor signals to the processing unit.

The sensor signal(s) may pertain to an intensity of light transmitted through the milk and/or reflected at the milk. The sensor signal(s) may (each) be based on light emission and/or detection at one of the at least one specific wavelength.

In other words, the sensor signal(s) may correlate to the intensity of light that is detected by the sensor unit, wherein the detected light has been reflected at and/or transmitted through the milk, and wherein the light is radiated and/or observed with respect to the at least one specific wavelength.

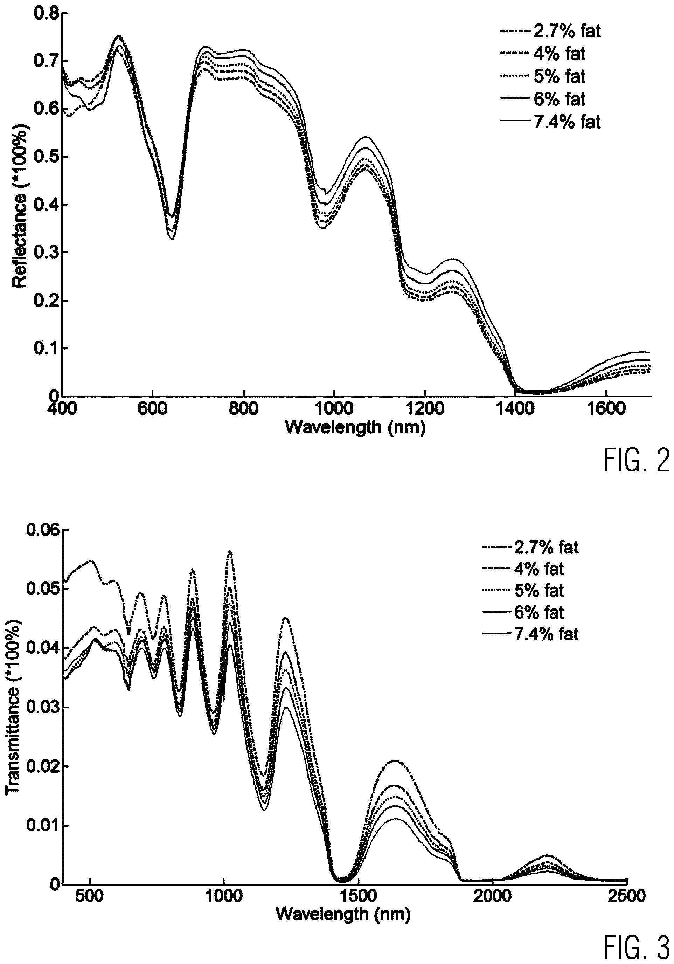

It has been found upon developing this invention that said intensity of light is in dependence of the component content of the milk, such as the fat content. For example, an increasing intensity can be measured in case of (light) transmittance when the milk contains increasingly less fat. This may be because the fat could block the light transmittance. A decreasing intensity can be measured in case of reflectance when the milk contains increasingly less fat. Reference is made to Figs. 2 and 3 reflecting those findings

It has also been found upon developing this invention that the intensity of light transmitted or reflected is in dependence of the wavelength of the light that is measured and/or emitted. As such, it is advantageous to limit detection of light, emission of light or both, to a specific wavelength or a range thereof. Again, reference is made to Figs. 2 and 3 reflecting this finding.

There may be certain wavelength ranges or values of wavelength, where the component content of the milk may be differentiated better. For example, there may be one wavelength range/value where the intensity of reflection of light is essentially independent from the fat content, e.g. at 680 nm and at 1500 nm, and there may be one other wavelength range/value where the intensity of reflection of light depends on the fat content, e.g. at approx. 525 nm and between 700 to 800 nm. As such, it is even better to focus detection of light, emission of light or both, to the at least one specific wavelength or another number of wavelengths (preferably two) so that the dependency of the intensity on the component content can be scaled, cf. Fig. 3 with regard to this finding.

The processing unit being adapted to determine the component content of the milk, preferably the milk contained in the reservoir, but also the above mentioned method, typically considers an intensity of detected light, i.e. within the sensor signal(s). The sensor signal(s) may contain or consist of a certain intensity value, typically as a function of the time. Upon electronically processing of the sensor signal(s), the processing unit may put one sensor signal pertaining to one specific wavelength and another sensor signals pertaining to another specific wavelength into a mathematical model, for example by adding the values or by making a division in order to get a result. The result and/or the intensity value may be continuously compared to a reference chart or reference function in order to make an estimation of the component content of the milk.

It may be an option to adopt more sophisticated mathematical methods, e.g. a fourier transformation, in order to determine the component content of the milk.
- Fig. 1: is a schematic sectional view of an embodiment of the apparatus;
- Fig. 2: is a plot of a transmittance as a function of the wavelength of light;
- Fig. 3: is a reflectance as a function of the wavelengths of light;
- Fig. 4: shows the fat content in the milk extracted from a human breast as a function of the percentage of milk extraction from the breast during a milk expression session of e.g. 15 minutes.

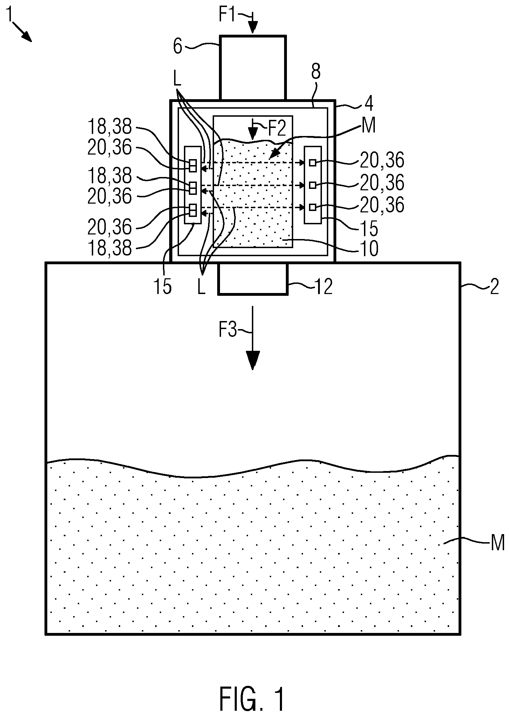

Fig. 1 shows a human milk handling apparatus 1. The apparatus 1 comprises a milk container 2 to accumulate human milk (in short: milk) that is indicated with reference mark M therein, a measuring device 4 coupled to the milk container 2, e.g. by means of a thread and/or a clamping means, and a connector 6 for a suction source of a pump in the form of a breast pump (not shown).

In particular, the milk container 2 is in the form of a baby bottle.

The connector 6 may comprise a thread and/or a nozzle to be connected to the pump.

The connector 6 is particularly connected to the measuring device 4 and forms the only entering path for the milk M into the milk container 2. The measuring device 4 may be detached from the milk container 2, e.g. by unscrewing or declamping, so that the connector 6 is detached inherently therewith.

In another (not shown) embodiment the connector 6 may be connected to the milk container 2 to form an entering path for the milk M into the milk container 2 independent from the particularly detachable measuring device 4.

The connector 6 may be detachable from the milk container 2 or the measuring device 4, for example by means of a thread or the like.

The measuring device 4 comprises a measuring head 8 with a reservoir 10 to accumulate milk M.

In Fig. 1, the reservoir 10 is a part of the apparatus 1.

In another case (not shown), the reservoir 10 is a part of a pump, such as a breast pump or a milk pump, where thus an apparatus 1 according to the invention may not necessarily have its own reservoir 10, but the pump provides the reservoir. A conduit of the pump or connected thereto may have the human milk M where the apparatus 1 can analyze the human milk M therein.

Referring back to Fig. 1, the reservoir 10 may serve to accumulate up to 5 ml of milk.

The milk container 2 may serve to accumulate up to 200 ml, particularly up to 250 ml, of milk.

At the connector 6 milk M collected from a lactating mother, especially collected by means of the pump (not shown) and/or its suction source, may enter along path F1 into the apparatus 1. The milk M may follow path F2 to the reservoir 10. In the reservoir 10, the milk M may accumulate until a certain milk volume is reached in the reservoir 10. The milk M may follow path F3 upon entering the milk container 2 through an outlet 12. The milk M may accumulate in the milk container 2.

At the outlet 12 there may be a valve such as a valve that is sensitive to static pressure.

The outlet 12 may be part of the measuring device 4 or the container 2.

The measuring head 8 further comprises a sensor unit 15 for emitting light L towards the reservoir 10 and for detecting the light L, and a processing unit. The processing unit is adapted to determine a component content, such as fat content, of the milk M contained in the reservoir 10 and is connected to the sensor unit 15.

The sensor unit 15 is adapted to emit and detect the light L at two specific wavelengths.

The first specific wavelength (of the two specific wavelengths) amounts to 940 nm ± 1 % and is different from the second specific wavelength (of the two specific wavelengths) that amounts to 800 nm ± 1 %. The sensor unit 15 is as well adapted to emit and detect the light L at more than the two specific wavelengths.

The first specific wavelength and the second specific wavelength both represent wavelengths of emission and wavelengths of detection. Thus, at each of the specific wavelengths the wavelength of emission and the wavelength of detection are the same.

It is particularly understood that emission/detection at the specific wavelengths means that at least 50 %, 75 %, or particularly 90%, of the emission/detection takes place at the given specific wavelength, e.g within the range 792 nm to 808 nm for 800 nm ± 1%.

The sensor unit 15 surrounds the reservoir 10 in order to conduct a measurement by reflectance of light L at the reservoir 10 and/or the milk and by transmittance of light L through the reservoir 10 and/or the milk M.

The sensor unit 15 may emit light L to be transmitted through the milk M and/or the reservoir 10 and reflected by the milk M and/or the reservoir, where said light L may be detected by the sensor unit 15.

The volume, amount, or level of the milk M in the reservoir 10 may be estimated, especially since the emitters 18, 38 and detectors 20, 36 placed at a certain level relative to the reservoir 10, in this case at three different levels each of which pertain to a fill level of milk in the reservoir 10.

The milk M may be estimated in terms of its composition, e.g. with respect to the fat content of the milk M. A measured transmittance and/or reflectance at the reservoir M containing the milk M with the fat content to be measured is highly dependent on the fat content of the milk. Thus, the fat content may be estimated by means of the processing unit handling information from the sensor unit 15.

The sensor unit 15 comprises three light emitters 18, 38 which emit light L at the two specific wavelengths. Each light emitter 18, 38 may therefor comprise for example two emitting means meant to each emit one of the two specific wavelengths so that both specific wavelengths are covered in terms of emission.

The sensor unit 15 comprises six light detectors 20, 36 which detect light L at the two specific wavelengths. Each light detector 20, 36 may comprise for example two detector means meant to each detect one specific wavelength so that both specific wavelengths are covered in terms of detection.

The two specific wavelengths are above 400 nm and below 1100 nm.

One of the specific wavelengths (940 nm) is contained in a first range between 920 nm and 960 nm. One of the specific wavelengths (800 nm) is contained in a second range between 780 nm and 820 nm.

Three of the light emitters 18, 38 are reflectance light emitters 38. Three of the light detectors 20, 36 are reflectance light detectors 36. The reflectance light emitters 38 and the reflectance light detectors 36 are arranged on the same side of the reservoir in order to realize a reflection of the light L at the the milk M.

Three of the light emitters 18, 38 are transmittance light emitters 18. Three of the light detectors 20, 36 are transmittance light detectors 20. The transmittance light emitters 18 and the transmittance light detectors are arranged on opposing sides of the reservoir 10 in order to realize a transmittance of the light L at though the milk M.

The processing unit is adapted to determine the fat content of the milk M. It is further possible to determine macro nutrients and/or components of the milk upon according configuration, e.g. by testing with reference milk M where such macro nutrients and/or components of the reference milk M are known.

By means of the apparatus 1 a method for analyzing the milk M may be conducted. In this method light L is emitted towards the human milk M, the light L is reflected at and/or transmitted through the human milk M, and the light L is detected. The emission and/or the detection of the light L may take place at at least one specific wavelength or at the at least two or more specific wavelengths as above. In the method light L is introduced at the two specific wavelengths into the reservoir 10 and/or transmitted towards the reservoir 10 at the two specific wavelengths.

Sensor signals resulting from a reflectance measurement or a transmissive measurement or a combination thereof may be obtained. The sensor signals may be electronically processed to output information of at least one component content of the milk M, particularly by means of the processing unit.

Fig. 2 shows experimental results within a plot that elucidates the correlation of the transmittance of light L through milk M, where the milk M has been evaluated considering specimens of milk M with five different contents of fat. Light L has been emitted and detected at a wavelength in the range 0 to 2500 nm. Fig. 3 shows experimental results gathered in a similar setup as with Fig. 2, where in Fig. 3 the reflectance of the light L is plotted over the wavelength range from 400 nm to approx. 1700 nm where accordingly the light L has been emitted and detected across the range of wavelength.

It can be seen from Fig. 2 that with increasing fat content the transmittance of the light L through milk M at least substantially reduces. It can also be seen that the transmittance of the light L is a function of the wavelength of the light L. For example, at approx. 1250 nm and approx. 1600 nm the transmittance scales more with fat content compared to e.g. 2000 nm, where at 2000 nm the transmittance is essentially zero at all fat contents. Another example is that at approx. 800 ± 10 nm and at approx. 940 nm the transmittance scales comparably well over the fat content, so that a consideration of both these wavelengths may be useful for the determination of the component content of the milk M.

Although it may look like from Fig. 2 that the best results may be gathered upon considering the wavelengths of approx. 1250 nm and approx. 1600 nm, the disadvantage here is that the cost of an appropriate sensor unit 15 is likely highly uneconomical; a sensor unit 15 to emit and/or detect below 1100 nm seems more preferred.

It can be seen from Fig. 3 that with increasing fat content the reflectance of the light L at the milk M at least substantially increases. It is noted that the effect seen from a varying fat content is thus the opposite relative to the transmittance of Fig. 2.

It can also be seen from Fig. 3 that the reflectance of the light L is a function of the wavelength of the light L. For example, at approx. 1000 nm and approx. 1200 nm the reflectance scales more with fat content compared to e.g. 1500 nm, where at 1500 nm the reflectance is essentially zero at all fat contents. Another example is that at approx. 525 ± 25 nm and at approx. 750 ± 25 nm the reflectance scales comparably well over the fat content, so that a consideration of both these wavelengths may be useful for the determination of the component content of the milk M.

The plots of Fig. 2 and 3 represent information that could be used to estimate the fat content. For example, when the sensor signal(s) represent a transmittance (i.e. intensity of transmitted light L through the milk M), the reduction of the sensor signal(s) over time may be understood as an increase of the fat content. The fat content may as well be estimated based on such experimental information.

When using the apparatus 1 of Fig. 1 with a pump, such as a breast pump to express human milk M from a lactating mother, in practice, at the beginning of the pumping session the collected milk M can be very translucent/watery. The milk M gets usually significantly less translucent/watery during the pumping session. The sensor unit 15 may continuously emit light L though the milk M in the reservoir 10 and detect the light L, where then such a change in fat content will have an effect on the intensity of the light L detected, which will translate to the sensor signal correlating therewith. The apparatus 1 continuously estimates the component content, in this case the fat content.

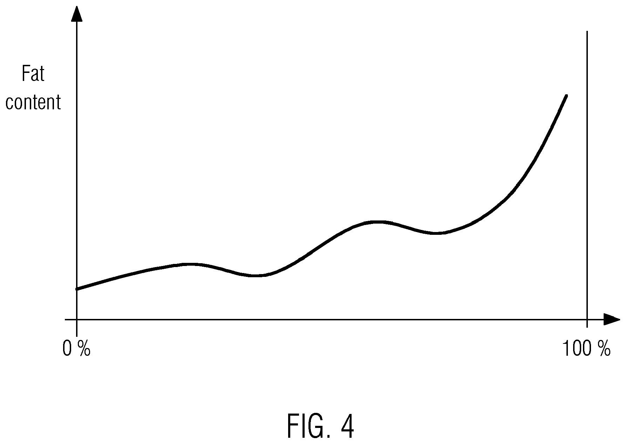

Fig. 4 shows a diagram with fat content on the vertical axis and the relative time of one milk expression cycle on the horizontal axis. It can be seen that fat content rises over time.

The information on the fat content may be used to extrapolate an information on the milk that has been removed. It is known that at the beginning of milk expression the fat content is lower and it goes up during expression (see figure 4). Based on this, the fat content provides an information on the milk removed from the breast and thus the milk still remaining and possibly to be removed from the breast (taking into account that 100% removal is not feasible). The information on the fat content in the milk and thus the removed milk percentage and consequently the milk still remaining to be removed may be used to drive the pump, e.g. to stop pump operation once a milk removal e.g. of 50 or 80 or 90 or 95% has been reached.

### List of Reference Signs

- 1: apparatus
- 2: milk container
- 4: measuring device
- 6: connector
- 8: measuring head
- 10: reservoir
- 12: outlet
- 15: sensor unit
- 18: light emitter
- 20: light detector
- 36: light detector
- 38: light emitter

- F1: path
- F2: path
- F3: path
- M: milk

## Claims

1. Human milk handling apparatus (1) comprising a measuring device (4) that has a sensor unit (15) for emitting light (L) towards human milk (M) and for detecting light reflected by or transmitted through the human milk (M); and comprising a processing unit which is adapted to determine at least a component content of said human milk (M) and connected to said sensor unit (15), said sensor unit (15) being adapted to emit and/or detect the light (L) at at least one specific wavelength.

2. The apparatus (1) according to claim 1, **characterized in that** the at least one specific wavelength comprises a wavelength of emission and a wavelength of detection which are at least substantially the same.

3. The apparatus (1) according to one of the preceding claims, **characterized in that** the sensor unit (15) comprises at least one light emitter (18, 38) arranged to emit light (L) at at least one specific wavelength.

4. The apparatus (1) according to one of the preceding claims, **characterized in that** the sensor unit (15) comprises at least one light detector (20, 36) arranged to detect light (L) at at least one specific wavelength.

5. The apparatus (1) according to one of the preceding claims, **characterized in that** the at least one specific wavelengths is above 400 nm and/or below 1100 nm.

6. The apparatus (1) according to one of the preceding claims, **characterized in that** the at least one specific wavelength is contained in a first range between 920 nm and 960 nm and/or in a second range between 750 nm and 800 nm and/or in a third range between 1040 nm and 1090 nm.

7. The apparatus (1) according to one of the preceding claims, **characterized in that** one of the at least one light emitter (18, 38) is a reflectance light emitter (38) and one of the at least one light detector (20, 36) is a reflectance light detector (36), both of which are arranged on the same side of the human milk (M).

8. The apparatus (1) according to one of the preceding claims, **characterized in that** one of the at least one light emitter (18, 38) is a transmittance light emitter (18) and one of the at least one light detector (20, 36) is a transmittance light detector (20), both of which are arranged on opposing sides of the human milk (M).

9. The apparatus (1) according to one of the preceding claims, **characterized in that** the processing unit is adapted to determine at least one of the following component contents:
- macro nutrients,
- fat content,
- protein content,
- components of the human milk (M).

10. The apparatus (1) according to one of the preceding claims, **characterized in that** the measuring device (4) is adapted to determine an amount of human milk (M), particularly in a reservoir (10) of the apparatus (1).

11. Breast pump built to express human milk (M) from a lactating mother, **characterized in that** the breast pump has the apparatus (1) according to one of claims 1 to 10 in order to determine a component content of said human milk (M).

12. A method for analyzing human milk (M) in which method light (L) is emitted towards the human milk (M), wherein said light (L) is reflected at and/or transmitted through the human milk (M), and wherein said reflected or transmitted light (L) is detected, wherein the emission of light (L) and/or the detection of the reflected or transmitted light (L) takes place at at least one specific wavelength.

13. The method according to claim 12, **characterized in that** the at least one specific wavelength contains a wavelength of transmission and a wavelength of detection which are are at least substantially the same.

14. The method according to any of claims 12 through 13, **characterized in that** sensor signals resulting from a reflectance measurement or a transmissive measurement or a combination thereof are obtained, which are electronically processed to output information of at least one component content of the human milk (M).

15. The method according to any of claims 12 through 14, **characterized in that** the component content of the human milk (M) is selected from the group comprising or consisting of:
- macro nutrients,
- fat content,
- protein content,
- components of the human milk (M).
